# FASCICULE DE BREVET EUROPEEN

(11) **EP 1 024 764 B1**
(45) Date de publication et mention de la délivrance du brevet: **22.08.2001**
(21) Numéro de dépôt: 98947707.0
(22) Date de dépôt: 21.10.1998
(51) Int. Cl.: A61F 2/00

(54) **IMPLANT PROTHETIQUE OBTURATEUR DE CANAL ANATOMIQUE, ET ENSEMBLE D'OBTURATION LE COMPORTANT**
PROTHESENIMPLANTAT ZUM VERSCHLIESSEN VON KÖRPERKANÄLEN SOWIE MIT DIESEM IMPLANTAT AUSGESTATTETE VERSCHLUSSEINHEIT
PROSTHETIC IMPLANT FOR OBSTRUCTING AN ANATOMICAL DUCT, AND OBSTRUCTING ASSEMBLY COMPRISING SAME

(30) Priorité: 22.10.1997 FR 9713464
(43) Date de publication de la demande: 09.08.2000
(73) Titulaire: SOFRADIM PRODUCTION, 01600 Trévoux (FR)
(72) Inventeur: ORY, François, Régis, F-69270 Fontaines-Saint-Martin (FR); THERIN, Michel, F-69002 Lyon (FR)
(74) Mandataire: Guerre, Dominique
(86) Numéro de dépôt international: IB9801675
(87) Numéro de publication internationale: WO9920204

(56) Documents cités:
- EP-A- 0 544 485
- EP-A- 0 614 650
- WO-A-92/06639
- WO-A-94/17747
- WO-A-95/32687

## Description

La présente invention concerne un implant prothétique obturateur de canal, de cavité ou d'orifice anatomique, et sera plus particulièrement décrit à titre d'exemple pour une utilisation dans le traitement des hernies de l'aine.

Différents implants prothétiques obturateurs d'orifice herniaire sont connus dans l'art antérieur sous la forme d'éléments textiles, bandelettes ou rectangles, enroulés pour former soit un cylindre, soit un cône. De tels éléments sont ensuite introduits dans l'orifice à obturer. Un certain nombre de solutions techniques ont été décrites, notamment dans les documents US-A-5 356 432, US-A-5 116 357, US-A-5 147 374, et US-A-5 456 720. Elles ont toutes en commun le préformage, par exemple par thermoformage, de ces cônes ou cylindres.

Par exemple, conformément à la figure 17 du document EP-A-0 544 485, on a décrit un implant prothétique obturateur de canal, de cavité, ou d'orifice anatomique, comprenant un élément textile poreux, en une seule plaque, obtenu à partir d'un tissu prothétique.

Dans une conformation à plat, non ramassée, cet élément poreux a la forme d'une pièce textile avec un bord ou pourtour extérieur continu. Il comporte plusieurs éléments radiaires de renfort, entrelacés dans la pièce textile, s'étendant et distribués autour d'une zone centrale exempte de tout dit élément. Ces éléments de renfort déterminent, par poussée locale dans ladite zone centrale, tout en contraignant de manière centripète le pourtour restant de l'élément textile, une conformation ramassée en volume.

Toutefois, ces solutions antérieures présentent plusieurs problèmes liés à ce préformage, notamment parce que les implants prothétiques qui en résultent sont souvent trop rigides. Cela se traduit par les inconvénients suivants :
- l'implant prothétique obturateur est traumatisant ou perforant vis à vis des structures péritonéales et ne peut s'adapter que difficilement à l'épaisseur de la paroi abdominale ;
- les formes en cône ou en cylindre de l'implant prothétique ne favorisent pas sa congruence à la géométrie complexe d'un orifice herniaire ;
- les implants prothétiques obturateurs sont soumis à un contact important avec les mains du chirurgien, augmentant ainsi le risque d'une infection post-opératoire ;
- les implants prothétiques obturateurs préformés traditionnels ne permettent pas au chirurgien d'atteindre des sites non accessibles au doigt.

La présente invention a pour objet un implant prothétique tel que défini précédemment, n'ayant pratiquement pas de forme privilégiée ou rémanente, donc gardant une bonne conformabilité, tout en offrant néanmoins une certaine rigidité lors de sa manipulation, d'une part pour être introduit sous forme ramassée dans l'orifice anatomique à obturer, et d'autre part pour s'appuyer sous cette forme contre le bord ou la paroi dudit orifice, le tout de manière non ou peu traumatisante.

Un implant prothétique selon la présente invention combine les caractéristiques suivantes :
- c'est dans l'élément textile que s'inscrivent et sont compris en continuité, et la zone centrale exempte de tout élément radiaire, et la zone périphérique qui comporte ces éléments radiaires ; la zone centrale est donc comblée par l'élément textile lui-même, et n'est donc pas occupée par un autre élément ou pièce, notamment rapporté ;
- l'élément textile comporte au moins deux plaques en tissu prothétique superposées ;
- et les deux éléments radiaires ont la forme de deux cordons textiles respectivement, constitués chacun par une multiplicité de points liant, notamment par couture ou tricot, les deux dites plaques, avec au moins un fil.

Préférentiellement, mais de manière non exclusive, dans la conformation ramassée, les deux éléments radiaires déterminent au moins deux lobes creux opposés, ouverts ou fermés, formés par les deux plaques entre lesdits éléments radiaires. Cette conformation lobée aide à la consistance de l'élément textile dans sa conformation ramassée.

Un autre objet de la présente invention est un ensemble d'obturation comprenant un implant prothétique tel que précédemment décrit et un ancillaire de pose.

En effet, on a trouvé qu'un implant prothétique selon l'invention présentait l'avantage que le non préformage de l'implant en forme de cylindre ou de cône favorisait sa congruence à la géométrie complexe d'un orifice, canal ou cavité anatomique, par exemple herniaire.

De préférence, les éléments radiaires s'étendent radialement chacun depuis le bord extérieur de l'élément textile jusqu'à un point intermédiaire entre un centre géométrique dudit élément textile et le bord extérieur dudit élément textile.

Plus préférentiellement, les éléments radiaires s'étendent chacun radialement jusqu'à la zone centrale.

Dans un mode d'exécution préféré, les éléments radiaires s'étendent chacun radialement sur une longueur limitée, à partir de et au voisinage du bord extérieur.

Préférentiellement, les deux plaques sont reliées l'une à l'autre par un surfilage discontinu le long des bords extérieurs des plaques, de manière à déterminer des lobes ouverts dans la conformation ramassée de l'implant.

Selon un mode d'exécution préféré les deux plaques sont reliées l'une à l'autre par les éléments radiaires, entretoisant lesdites plaques.

Dans un autre mode d'exécution préféré, l'élément textile comprend au moins trois plaques liées entre elles, de nature identique ou différente, l'une des plaques ayant éventuellement une structure tridimensionnelle.

De manière préférée, l'élément textile est susceptible d'être obtenu par découpe d'une pièce circulaire rassemblant une plaque extérieure, une plaque intermédiaire, et une plaque intérieure, par pliage de ladite pièce selon un diamètre en deux flancs en vis-à-vis, des éléments radiaires reliant les trois plaques entre elles, et au moins deux éléments reliant les deux flancs en vis-à-vis de la plaque intérieure.

Plus préférentiellement encore, les éléments radiaires relient deux plaques l'une à l'autre, la troisième plaque étant reliée aux deux autres par le surfilage de son bord extérieur.

Dans un mode d'exécution préféré, l'implant comporte une plaque complémentaire de recouvrement, liée à l'élément textile, ayant une forme différente de ce dernier et adaptée avec une fente appropriée pour entourer tout conduit anatomique.

La présente invention sera mieux comprise par la description détaillée des modes d'exécution préférés donnés à titre d'exemple uniquement, et en se référant au dessin en annexe dans lequel :
- **la Figure 1** représente une vue en plan d'un implant prothétique obturateur selon l'invention, dans sa conformation non ramassée à plat ;
- **la Figure 2** représente une variante de l'implant prothétique de la Figure 1, vue de dessous avec un élément textile présentant trois plaques, dans sa conformation non ramassée à plat ;
- **la Figure 3** représente une plaque complémentaire de recouvrement pour l'implant prothétique des Figures 1 et 2 ;
- **la Figure 4** représente une vue en plan, dans sa conformation non ramassée, à plat, d'un autre mode d'exécution préféré de l'implant prothétique selon l'invention telle qu'illustrée par les Figures 1 et 2, muni de la plaque complémentaire de la Figure 3 ;
- **la Figure 5** représente la vue du dessous de l'implant prothétique selon la Figure 4 ;
- **la Figure 6** représente une vue en plan d'un autre mode d'exécution préféré de l'implant prothétique selon l'invention, dans sa conformation non ramassée, à plat, dans un ensemble ancillaire d'obturation ;
- **la Figure 7** représente une vue de côté de l'ensemble selon la Figure 6 ;
- **la Figure 8** représente une vue en plan de l'implant prothétique de la Figure 7 selon la ligne A-A, après un commencement de plicature de celui-ci vers sa conformation ramassée ;
- **la Figure 9** représente une vue en perspective latérale de la Figure 8, après plicature complète de l'implant prothétique dans sa conformation ramassée ;
- **la Figure 10** représente une vue en plan d'un implant prothétique selon un autre mode d'exécution selon l'invention ;
- **la Figure 11** représente une vue de côté de l'implant prothétique de la Figure 10 ;
- **la Figure 12** représente une vue schématique de la manipulation d'un implant obturateur selon les figures 1 et 2 pour obturer un canal inguinal ;
- **la Figure 13** représente une vue schématique du pliage de l'implant selon les figures 1 et 2 et son introduction dans le canal inguinal ;
- **la Figure 14** représente une vue schématique du canal inguinal obturé avec un implant obturateur selon les figures 1 et 2 ;
- **la Figure 15** représente une vue schématique de la manipulation d'un implant obturateur selon les figures 3 à 5 pour obturer un canal inguinal, et protéger contre les récidives ;
- **la Figure 16** représente une vue schématique du pliage de l'implant selon les figures 3 à 5 et son introduction dans le canal inguinal ;
- **la Figure 17** représente une vue schématique du canal inguinal obturé avec un implant obturateur et une plaque de recouvrement complémentaire selon les figures 3 à 5.

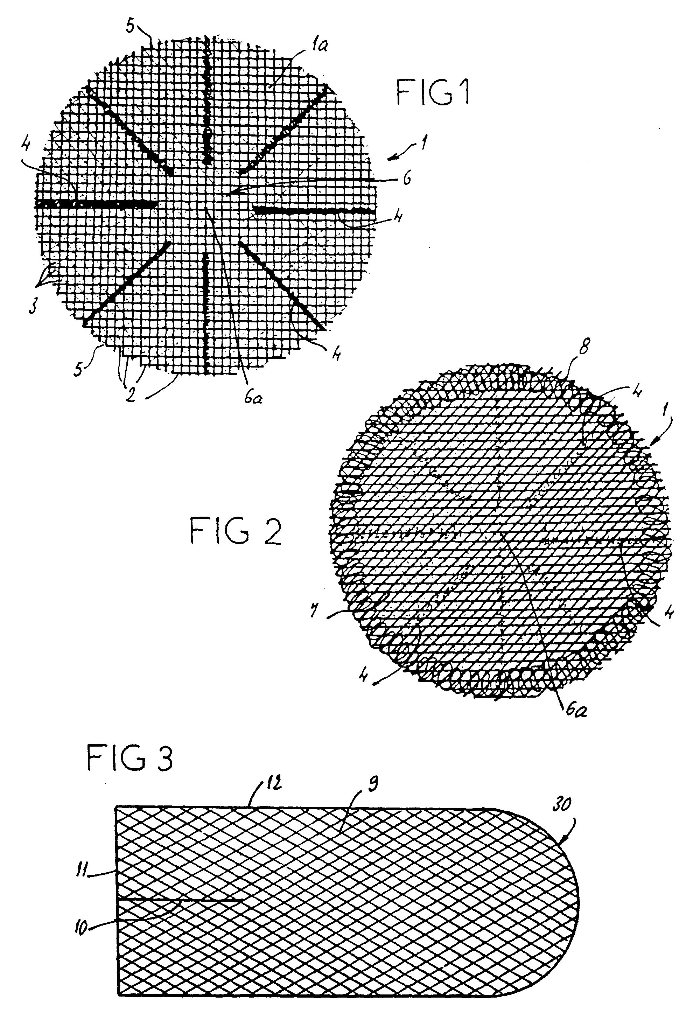

La figure 1 représente un implant prothétique obturateur 1 selon la présente invention, vu en plan de dessus. L'implant prothétique est de forme généralement circulaire, et présente par exemple un diamètre d'environ 8 cm. Cette forme peut bien entendu varier, et on peut utiliser par exemple des implants prothétiques de forme elliptique, présentant par exemple un diamètre compris entre environ 6,5 cm (petit diamètre), et 8,5 cm (grand diamètre).

L'implant prothétique obturateur 1, dans sa conformation non ramassée à plat, comprend un élément textile la poreux, ayant la forme d'une pièce avec un bord extérieur ou pourtour 5 continu, et est constitué par une plaque 2 (supérieure selon Fig. 1) et une deuxième plaque 3 (inférieure selon Fig. 1), superposées et composées chacune de fils tricotés de chaîne et de trame, en tissu prothétique relativement flexible, par exemple en polymère biocompatible non résorbable, et de préférence à base de fils multibrins en polyester 50 dtex. Un tel tissu prothétique est fabriqué et commercialisé par le demandeur sous la référence PAC, et consiste en un tricot à fils de chaîne tramés, à jours carrés, réalisé avec trois nappes de fils de chaîne. Le tissu présente une bonne stabilité bidirectionnelle et est ineffilochable à la découpe. Les deux plaques peuvent bien entendu être de même nature ou différente, mais présentent généralement chacune une structure bidimensionnelle quadrillée, par exemple avec des pores d'environ 1,5 mm x 1,5 mm. Comme on peut l'apercevoir sur la figure 1, par rapport au quadrillage de la structure bidimensionnelle, les deux plaques peuvent être décalées angulairement l'une par rapport à l'autre, et la plaque 3 est de préférence orientée à 45° par rapport à la plaque 2.

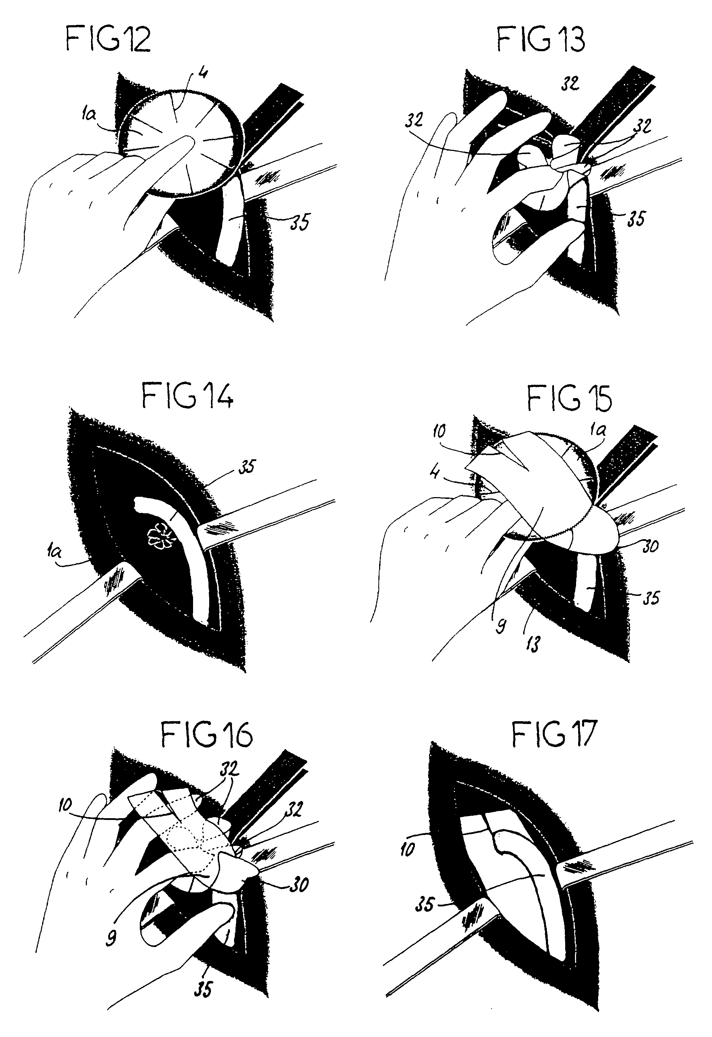

Ces deux plaques 2,3 sont reliées l'une à l'autre par des éléments 4 radiaires, et de guidage de plicature, s'étendant et distribués autour d'une zone centrale 6 exempte de tout dit élément radiaire. Comme cela sera mieux illustré par rapport à la figure 13, les éléments radiaires 4 déterminent, lorsqu'on applique une poussée locale, par exemple avec le doigt, dans la zone centrale 6, et tout en contraignant de manière centripète le pourtour restant de l'élément textile la, une conformation ramassée en volume comprenant au moins deux lobes creux 32 opposés, formés par les deux plaques 2 et 3 entre les éléments radiaires 4.

De préférence, ces éléments radiaires 4 sont intégrés dans l'élément textile, en entretoisant les deux plaques, et les liant l'une à l'autre. Chaque élément radiaire a la forme d'un cordon textile, constitué par une multiplicité de points de couture liant les plaques 2 et 3 avec au moins un fil, de préférence le même que celui ayant servi au tricotage du tissu prothétique précité. Comme représenté sur les figures 1 à 5, huit coutures radiales 4, angulairement équiréparties, partent du bord extérieur 5 des plaques, ou de son voisinage, et se dirigent vers la zone centrale 6 et le centre géométrique 6a du cercle, par exemple sur environ 3 cm. Si les plaques sont de forme elliptique, les coutures radiales 4 ont des longueurs inégales, et elles convergent vers le centre géométrique 6a de l'ellipse, s'arrêtant par exemple à environ 1 cm de celui-ci. Ainsi les éléments radiaires 4 ne s'étendent pas jusqu'au centre géométrique 6a de l'élément textile la, mais jusqu'à la zone centrale 6, ce qui évite le risque d'une perforation ou traumatisme des organes sensibles dans ou autour de l'orifice ou canal à obturer lorsque l'implant est dans sa conformation ramassée.

Comme le montrent bien les figures 1 et 2, c'est dans l'élément textile 1a que sont comprises et s'inscrivent en continuité, et la zone centrale 6 exempte de tout élément radiaire 4, et la zone adjacente et périphérique comprenant précisément ces éléments radiaires 4 ; de telle sorte qu'un implant selon la présente invention se réduit audit élément textile 1a avec ses éléments radiaires 4, en général intégrés

L'implant prothétique obturateur selon la figure 2 se distingue de celui représenté conformément à la figure 1, en ce que l'élément textile la comporte en plus une troisième plaque 7, de préférence à structure tridimensionnelle poreuse réhabitable, et présentant par exemple une épaisseur d'environ 1,5 à 2 mm. Une telle plaque est commercialisée par le demandeur, sous la dénomination PAT. Cette plaque est un tricot chaîne tridimensionnel à entretoise, présentant deux faces à jours hexagonaux liées par une entretoise de fils de chaîne, et qui lui confère une élasticité tridirectionnelle. La troisième plaque est celle qui reposera sur les différents organes lors de la réparation d'une hernie inguinale directe par exemple, et protègera ceux-ci d'un contact éventuellement traumatisant avec les plaques 2 et 3.

Les plaques 2 et 3 sont solidarisées à la troisième plaque 7 par un surfilage discontinu 8 le long du bord circulaire 5 commun desdites plaques. Le fil de surfilage est de préférence identique à celui utilisé pour les éléments radiaires 4.

L'ensemble obtenu conformément à la figure 2 peut être optionnellement enduit de collagène, par exemple bovin type 1.

La figure 3 illustre une plaque de recouvrement complémentaire pour l'implant prothétique selon l'invention. Cette plaque 9 peut présenter une largeur d'environ 4,5 cm et une longueur d'environ 11 cm, et est généralement de forme rectangulaire. Elle comporte une fente 10, adaptée à recevoir un conduit anatomique, par exemple les vaisseaux spermatiques en cas de réparation herniaire, et s'étendant depuis un bord droit intérieur 11, ou petit côté de la plaque 9, de préférence perpendiculaire au bord transversal 12, ou grand côté de la plaque 9 ; la fente 10 s'étend au milieu de la plaque, sur une longueur d'environ 3 cm. A l'opposé du bord extérieur arrondi 11 de la plaque 9 de recouvrement complémentaire, cette dernière comporte un autre bord extérieur 30.

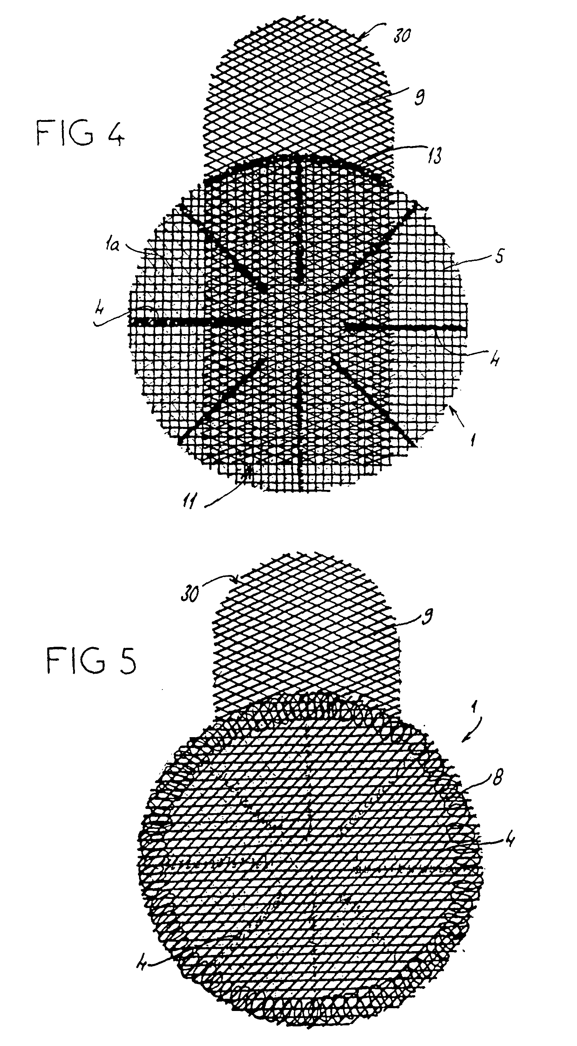

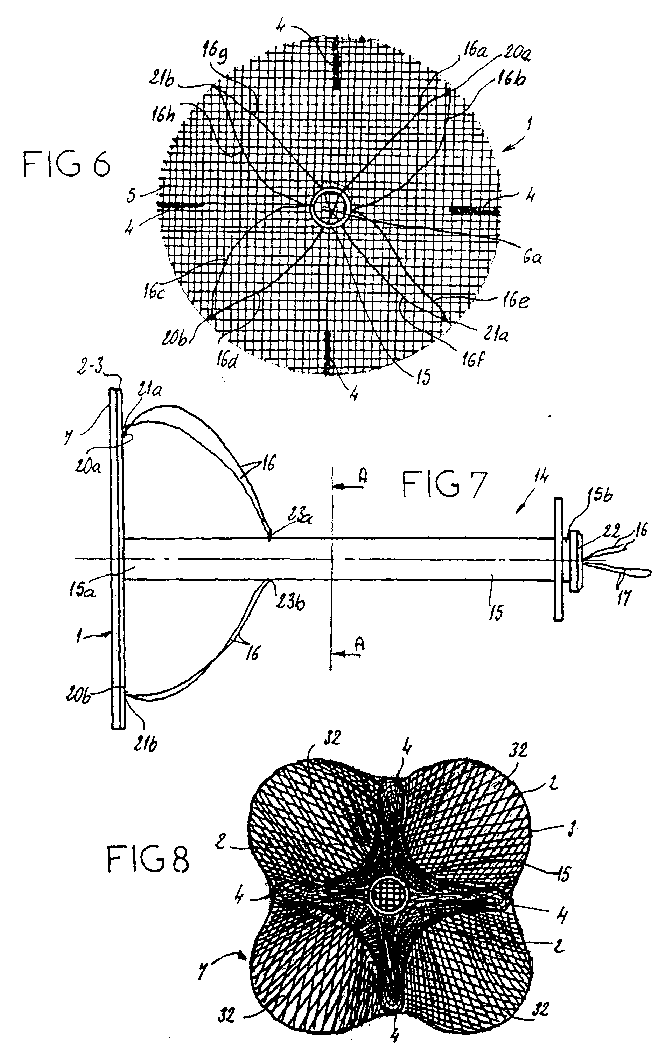

Comme illustré par les figures 4 et 5, qui montrent des vues respectivement en plan de dessus et dessous de l'implant prothétique selon l'invention, la plaque 9 complémentaire de recouvrement est fixée sur l'élément textile 1a, par exemple par sutures 13, en sorte que l'axe de symétrie longitudinale de ladite plaque coïncide avec un diamètre de l'élément textile circulaire la, avec le bord intérieur 11 compris dans le cercle défini par ledit élément textile, et le bord extérieur 30 à l'extérieur dudit cercle. La fixation est avantageusement réalisée le long du bord circulaire de l'élément textile la. La fonction de cette plaque de recouvrement complémentaire est de protéger les zones périphériques de l'orifice herniaire, potentiellement fragiles, des risques de récidive.

Comme dit précédemment, l'invention concerne également un ensemble d'obturation comprenant un implant prothétique obturateur 1 tel que décrit précédemment et un ancillaire de pose 14. Cette partie de l'invention est illustrée par les figures 6 à 9, dans lesquelles la seule différence par rapport à l'implant prothétique des figures précédentes est que les éléments radiaires 4 et de guidage de plicature, sont constitués par seulement quatre coutures distribuées radialement autour du centre géométrique 6a de l'implant obturateur, et s'étendant sur une distance plus courte vers la zone centrale 6. Dans le cas de la présente invention, un ancillaire de pose 14 peut être constitué d'un élément tubulaire rigide 15, par exemple une canule rigide, dans lequel est passé un fil 16 de traction, le fil 16 étant entrelacé librement dans la plaque 2 de l'élément textile 1, de sorte que les extrémités du fil 16, ainsi qu'une boucle 17 formée du fil 16 dépassent de l'extrémité proximale ouverte 15b de l'élément tubulaire 15. Le fil 16 sort de l'élément tubulaire 15 par des ouvertures 23a,23b à distance de l'extrémité distale ouverte 15a de l'élément tubulaire 15, cette distance correspondant par exemple au moins à la distance séparant le centre géométrique 6a de l'élément textile la de son bord extérieur 5, par exemple à au moins 4 cm (rayon du cercle de l'implant prothétique obturateur). Le fil 16 passe d'une ouverture 23a vers le bord extérieur 5 selon la référence 16a, et autour d'un fil de chaîne ou de trame à un endroit 20a de la plaque 2, pour revenir selon la référence 16b en direction d'un endroit diamétralement opposé 20b à celui précédemment décrit, soit en repassant vers les ouvertures 23a et 23b, soit directement selon la référence 16c pour retourner ensuite vers les ouvertures 23a,23b selon la référence 16d. Cette opération est recommencée avec le même fil 16 selon les références 16e,16f,16g et 16h, aux endroits 21a, 21b, de manière à former un motif croisé ; cf. figure 6.

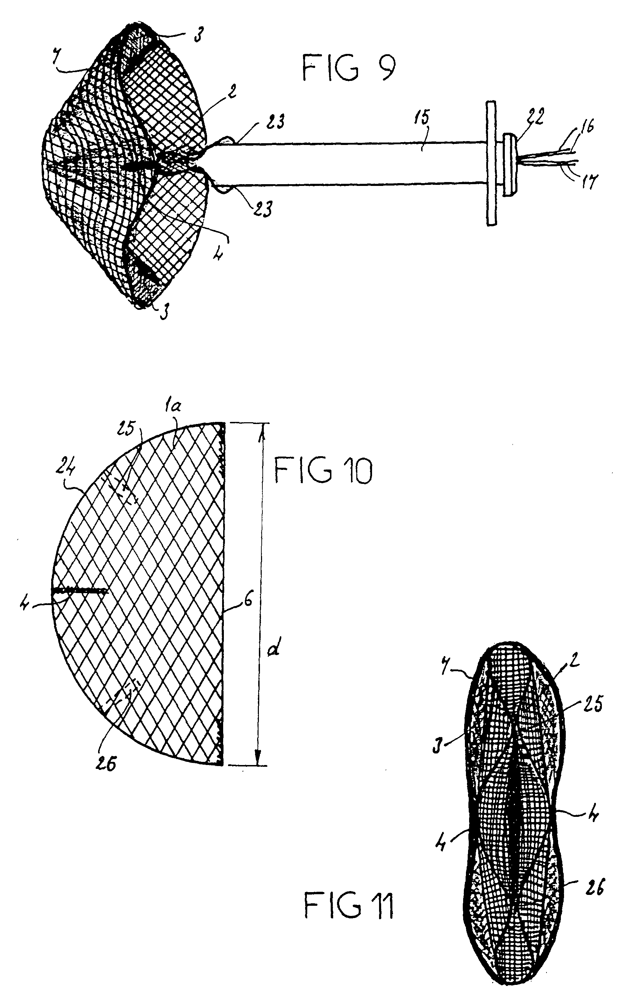

Pour plier l'implant prothétique 1 avant son introduction dans l'orifice inguinal, il suffit alors de tirer sur la boucle 17 et les extrémités du fil 16 à l'extrémité proximale ouverte 15b de l'élément tubulaire 15. Etant donné que le centre de l'extrémité ouverte 15b de l'élément 15 communique avec le centre géométrique 6a de l'implant prothétique 1, celui-ci va se plier selon une forme quadri-lobée 32 régulière correspondant à la vue représentée en figure 8, qui est une vue le long de la ligne A-A de la figure 7. On constate que l'implant prothétique 1 se plie selon l'entrelacement du fil, mais que cette plicature est également guidée par les éléments 4 de renfort et de guidage.

En poursuivant la traction sur le fil 16 et la boucle 17, on arrive à la conformation finale illustrée par la figure 9, c'est-à-dire que l'implant prothétique obturateur 1 présente une forme de rosette, prête à introduction dans le canal, orifice ou cavité anatomique, par le chirurgien. L'ancillaire de pose permet donc avec un seul fil, par exemple passé en boucle, de ramasser l'implant prothétique obturateur 1 autour de l'axe de l'élément tubulaire 15 par simple traction. Le retrait du fil 16 se fait en tirant sur la boucle 17 et libère ainsi l'implant dans son site. L'utilisation de cet ancillaire diminue le contact des doigts du chirurgien avec le corps, ainsi qu'avec l'implant, et permet d'atteindre des sites non accessibles au doigt tout seul.

Dans la conformation ramassée en rosette de l'implant prothétique obturateur 1, partielle selon Fig. 8, et totale selon Fig. 9, chaque lobe creux 32 est délimité entre une section en quart de cercle de la première plaque 2, de forme convexe extérieure, et une section en vis-à-vis en quart de cercle de la deuxième plaque 3, de forme concave intérieure, ces sections étant réunies par deux éléments radiaires 4, communs chacun à deux lobes 32 adjacents. Les quatre lobes 32 déterminent entre eux une forme interne en croix.

Les figures 10 et 11 représentent un autre mode d'exécution préféré de l'implant prothétique obturateur selon l'invention. Cet autre mode sera décrit uniquement par rapport aux différences avec les modes d'exécution des figures 1 à 5. L'implant obturateur 24 a une forme hémi-circulaire, dans sa forme non ramassée à plat, et l'élément textile la est obtenu par découpe d'une pièce circulaire rassemblant trois plaques, intérieure 2, intermédiaire 3, et extérieure 7 respectivement. Cette pièce est pliée en deux flancs en vis-à-vis selon un diamètre d de l'élément textile la, de manière a former un demi-cercle. Les trois plaques sont reliées par des éléments radiaires 4, et les plaques intermédiaire 3 et extérieure 7 reliées l'une à l'autre par un surfilage. La plaque intérieure 2 présente toutefois au moins deux éléments, par exemple des coutures 25,26 reliant ses deux flancs en vis-à-vis (cf. figure 11).

Lors de son utilisation, l'implant prothétique obturateur 1 est présenté en regard du canal ou de l'orifice, par exemple inguinal, tel que représenté schématiquement aux figures 12 à 17. Dans ces figures la référence 35 indique le cordon spermatique. L'implant est introduit dans l'orifice ou canal par pression sur la zone centrale 6 ou le centre géométrique 6a de la plaque 2 de l'élément textile la à l'aide d'un doigt, d'une pince ou de l'ancillaire de pose. Ainsi, l'implant passe, par le biais d'une contrainte centripète sur le pourtour restant de l'élément textile, qui peut être exercée par exemple par les parois de l'orifice inguinal, de la conformation à plat vers une conformation ramassée en volume, tel qu'illustré par les figures 13, 14 et 16 en particulier. En effet, par sa compliance, un implant prothétique selon l'invention "se moule" sur les structures anatomiques en rapport avec l'orifice ou canal à obturer, et forme au moins deux lobes creux opposés 32, les éléments radiaires 4 guidant la plicature et la formation des lobes, et conférant également à l'implant obturateur une certaine résistance radiale pour une meilleure tenue mécanique une fois implanté.

Les figures 15 à 17 montrent l'application de l'implant selon les figures 3 à 5, qui ne diffère de ce qui précède qu'en ce que la plaque complémentaire de recouvrement 9 est positionnée au-dessus de l'élément textile la, de manière à couvrir une zone anatomique autour de l'orifice inguinal, afin de prévenir des récidives éventuelles, la fente 10 entourant les vaisseaux spermatiques 35, comme on peut le voir sur la figure 17.

D'autres avantages, spécifiques pour la chirurgie herniaire, résultant de l'implant prothétique obturateur selon la présente invention, sont les suivants :
- lorsqu'une troisième plaque de structure tridimensionnelle est présente, en sorte de se situer à l'extérieur de l'implant dans sa conformation ramassée, cette structure tridimensionnelle extérieure est très compliante et douce ; c'est la seule structure au contact des tissus environnants, elle n'est pas traumatisante notamment pour les éléments du cordon spermatique et autorise une très bonne réhabitation ;
- les éléments radiaires, assurant la tenue mécanique de l'implant prothétique obturateur, ne sont pas au contact d'éléments critiques tels que le canal déférent, les vaisseaux spermatiques, iliaques ou fémoraux, les nerfs régionaux ;
- l'arrêt des éléments radiaires au niveau de la zone centrale permet à l'extrémité profonde de l'implant prothétique, dans sa conformation ramassée, une fois en place, de ne pas être traumatisante ou perforante vis à vis des structures péritonéales et de s'adapter à l'épaisseur de la paroi abdominale ;
- dans le mode d'exécution avec troisième plaque de structure tridimensionnelle, toutes les zones potentiellement fragiles sont protégées des récidives (orifice direct dans le cas d'un traitement de hernie indirecte et inversement) par un seul et même implant ;
- dans le mode d'exécution avec plaque de recouvrement complémentaire, solidaire de l'élément textile, la fixation aux tissus périphériques de celle-ci contribue à prévenir les risques de migration profonde dans l'implant prothétique ;
- la porosité très élevée de l'élément textile permet une réhabitation conjonctive à coeur pontant l'espace et comblant ainsi de façon stable l'orifice ;
- dans le mode d'exécution avec troisième plaque de structure tridimensionnelle, le surfilage permet avantageusement de ne pas écraser la plaque supérieure sur les plaques inférieures comme l'aurait fait une couture classique, et ainsi de préserver la structure tridimensionnelle compliante sur toute la surface extérieure utile de l'implant.

## Revendications

1. Implant prothétique obturateur (1) de canal, de cavité ou d'orifice anatomique, comprenant un élément textile (1a) poreux, obtenu à partir d'un tissu prothétique, ayant dans une conformation à plat, non ramassée, la forme d'une pièce avec un bord ou pourtour extérieur (5) continu, et ledit élément comportant au moins deux éléments (4) radiaires, s'étendant et distribués autour d'une zone centrale (6) exempte de tout élément radiaire, et lesdits éléments radiaires déterminant, par poussée locale dans ladite zone centrale (6), tout en contraignant de manière centripète le pourtour restant de l'élément textile (1a), une conformation ramassée en volume, **caractérisé en ce que**, en combinaison, d'une part l'élément textile (1a) dans lequel s'inscrivent en continuité, et ladite zone centrale, et une zone périphérique comprenant lesdits éléments radiaires, comporte au moins deux plaques (2,3) en tissu prothétique superposées, et d'autre part les deux éléments (4) radiaires ont la forme de deux cordons textiles respectivement, constitués chacun par une multiplicité de points liant les deux plaques (2,3) avec au moins un fil.

2. Implant selon la revendication 1, caractérisé en ce que, dans la conformation ramassée, les deux éléments radiaires (4) déterminent au moins deux lobes (32) creux opposés, ouverts ou fermés, formés par les deux plaques (2,3) entre lesdits éléments radiaires (4).

3. Implant selon la revendication 1, caractérisé en ce que les éléments radiaires (4) s'étendent radialement chacun depuis le bord extérieur (5) de l'élément textile jusqu'à un point intermédiaire entre un centre géométrique (6a) dudit élément textile et le bord extérieur (5) dudit élément textile.

4. Implant selon la revendication 1, caractérisé en ce que les éléments radiaires (4) s'étendent chacun radialement jusqu'à la zone centrale (6).

5. Implant selon la revendication 3, caractérisé en ce que les éléments radiaires (4) s'étendent chacun radialement sur une longueur limitée à partir de et au voisinage du bord extérieur (5).

6. Implant selon la revendication 1, caractérisé en ce que les deux plaques (2,3) sont reliées l'une à l'autre par un surfilage (8) discontinu le long des bords extérieurs (5) des plaques.

7. Implant selon la revendication 1, caractérisé en ce que les éléments (4) radiaires entretoisent les plaques (2,3).

8. Implant selon la revendication 1, caractérisé en ce que l'élément textile (1a) comprend au moins trois plaques (2,3,7) liées entre elles, de nature identique ou différente, l'une des plaques ayant éventuellement une structure tridimensionnelle.

9. Implant selon la revendication 8, caractérisé en ce que l'élément textile (1a) est susceptible d'être obtenu par découpe d'une pièce circulaire rassemblant une plaque extérieure (7), une plaque intermédiaire (3), et une plaque intérieure (2), par pliage de ladite pièce selon un diamètre (d) en deux flancs en vis-à-vis, des éléments (4) radiaires reliant les trois plaques entre elles, et au moins deux éléments (25,26) reliant les deux flancs en vis-à-vis de la plaque intérieure (2).

10. Implant selon la revendication 8, caractérisé en ce que les éléments radiaires (4) relient deux plaques l'une à l'autre, la troisième plaque (7) étant reliée aux deux autres (2,3) par le surfilage de son bord extérieur (5).

11. Implant selon la revendication 1, caractérisé en ce qu'il comporte une plaque complémentaire (9) de recouvrement, liée à l'élément textile (1a), ayant une forme différente de ce dernier et adaptée avec une fente appropriée pour entourer tout conduit anatomique.

12. Ensemble d'obturation de canal, de cavité ou d'orifice anatomique, comprenant un implant obturateur selon l'une quelconque des revendications 1 à 11, et un ancillaire de pose (14).

## Patentansprüche

1. Prothetisches Verschlußimplantat (1) zum Verschließen eines anatomischen Kanals, Hohlraums oder einer anatomischen Öffnung, mit einem porösen textilen Element (1a), das aus einem Prothesengewebe gefertigt ist, das in einem nicht gerafften flach ausgebreiteten Zustand die Form eines Stücks mit einem durchgehenden äußeren Rand oder Umfang (5) aufweist, wobei das Element zumindest zwei strahlenförmige Elemente (4) aufweist, die sich um einen mittigen Bereich (6), der von den strahlenförmigen Elementen frei ist, erstrecken und verteilt sind, und wobei die strahlenförmigen Elemente bei lokalem Druck in dem mittigen Bereich (6), wodurch der übrige Umfang des textilen Elements (1a) zentripetal eingezwängt wird, einen im Volumen gerafften Zustand bestimmen, dadurch gekennzeichnet, daß, in Kombination, einerseits das textile Element (1a), in den sich in Kontinuität sowohl der mittige Bereich als auch ein umliegender Bereich, der die strahlenförmigen Elemente aufweist, einbeschreibt, zumindest zwei übereinander angeordnete Lagen (2, 3) aus Prothesengewebe aufweist, und daß andererseits die beiden strahlenförmigen Elemente (4) die Form von zwei textilen Schnüren aufweisen, die jeweils durch eine Vielzahl von Stellen gebildet sind, die die beiden Lagen (2, 3) mit zumindest einem Faden verbinden.

2. Implantat nach Anspruch 1, dadurch gekennzeichnet, daß im gerafften Zustand die beiden strahlenförmigen Elemente (4) zumindest zwei gegenüberliegende, offene oder geschlossene, hohle Lappen (32) bestimmen, die durch die beiden Lagen (2, 3) zwischen den strahlenförmigen Elementen (4) gebildet sind.

3. Implantat nach Anspruch 1, dadurch gekennzeichnet, daß die strahlenförmigen Elemente (4) sich jeweils radial von dem äußeren Rand (5) des textilen Elements bis zu einer Zwischenstelle zwischen einem geometrischen Mittelpunkt (6a) des textilen Elements und dem äußeren Rand (5) des textilen Elements erstrecken.

4. Implantat nach Anspruch 1, dadurch gekennzeichnet, daß die strahlenförmigen Elemente (4) sich jeweils radial bis zu dem mittigen Bereich (6) erstrecken.

5. Implantat nach Anspruch 3, dadurch gekennzeichnet, daß die strahlenförmigen Elemente (4) sich jeweils von dem äußeren Rand (5) oder von diesem benachbart radial über eine begrenzte Länge erstrecken.

6. Implantat nach Anspruch 1, dadurch gekennzeichnet, daß die beiden Lagen (2, 3) miteinander durch einen diskontinuierlichen Überkantstich (8) entlang der äußeren Ränder (5) der Lagen verbunden sind.

7. Implantat nach Anspruch 1, dadurch gekennzeichnet, daß die strahlenförmigen Elemente (4) die Lagen (2, 3) durchdringen.

8. Implantat nach Anspruch 1, dadurch gekennzeichnet, daß das textile Element (1a) zumindest drei untereinander verbundene Lagen (2, 3, 7) von identischer oder unterschiedlicher Beschaffenheit aufweist, wobei eine der Lagen gegebenenfalls eine dreidimensionale Struktur aufweist.

9. Implantat nach Anspruch 8, dadurch gekennzeichnet, daß das textile Element (1a) in der Lage ist, durch Zuschnitt eines kreisförmigen Stücks erhalten zu werden, das durch Falten des Stückes um einen Durchmesser (d) zu zwei gegenüberliegenden Seiten eine äußere Lage (7), eine mittlere Lage (3) und eine innere Lage (2) zusammenfügt, wobei die strahlenförmigen Elemente (4) die drei Lagen untereinander verbinden, und zumindest zwei Elemente (25, 26) die beiden gegenüberliegenden Seiten der inneren Lage (2) verbinden.

10. Implantat nach Anspruch 8, dadurch gekennzeichnet, daß die strahlenförmigen Elemente (4) zwei Lagen miteinander verbinden, wobei die dritte Lage (7) mit den beiden anderen (2, 3) durch den Überkantstich ihres äußeren Randes (5) verbunden ist.

11. Implantat nach Anspruch 1, dadurch gekennzeichnet, daß es eine komplementäre Decklage (9) aufweist, die mit dem textilen Element (1a) verbunden ist, und die eine von letzterem unterschiedliche Form aufweist und mit einem geeigneten Schlitz zum Umgeben eines anatomischen Leiters versehen ist.

12. Verschlußanordnung zum Verschließen eines anatomischen Kanals, Hohlraumes oder einer anatomischen Öffnung, mit einem Verschlußimplantat gemäß einem der Ansprüche 1 bis 11, und mit einer Anbringhilfe (14).

## Claims

1. A prosthetic implant (1) for obturating an anatomical duct, cavity or orifice, comprising a porous textile element (1a) obtained from a prosthetic fabric, having in a flat and nongathered-up configuration the shape of a patch with a continuous outer edge or periphery (5), and the said element comprising at least two radial elements (4), extending and distributed around a central zone (6) free from any radial element, and said radial elements determining, by local thrust in the said central zone (6), whilst centripetally stressing the remaining periphery of the textile element (1a), a configuration gathered up in volume, characterized in that, in combination, on the one hand the textile element (1a), in which are located without break both the said central zone and a peripheral zone comprising the said radial elements, comprises at least two superimposed panels (2, 3) of prosthetic fabric and, on the other hand, the two radial elements (4) are shaped like two textile strips respectively, each consisting of a multiplicity of stitches linking the two panels (2, 3) with at least one thread.

2. Implant according to Claim 1, characterized in that, in the gathered-up configuration, the two radial. elements (4) determine at least two opposite hollow, open or closed lobes (32) formed by the two panels (2, 3) between the said radial elements (4).

3. Implant according to Claim 1, characterized in that the radial elements (4) each extend radially from the outer edge (5) of the textile element to an intermediate point between a geometric centre (6a) of the said textile element and the outer edge (5) of the said textile element.

4. Implant according to Claim 1, characterized in that the radial elements (4) each extend radially up to the central zone (6).

5. Implant according to Claim 3, characterized in that the radial elements (4) each extend radially over a limited length, starting from and in the vicinity of the outer edge (5).

6. Implant according to Claim 1, characterized in that the two panels (2, 3) are connected to each other by discontinuous overstitching (8) along the outer edges (5) of the panels.

7. Implant according to Claim 1, characterized in that the radial elements (4) tie the panels (2, 3).

8. Implant according to Claim 1, characterized in that the textile element (1a) comprises at least three panels (2, 3, 7) bound to each other, of identical or different nature, one of the panels possibly having a three-dimensional structure.

9. Implant according to Claim 8, characterized in that the textile element (1a) is capable of being obtained by cutting out a circular patch bringing together an outer panel (7), an intermediate panel (3) and an inner panel (2), by folding the said patch along a diameter (d) with two sides facing each other, radial elements (4) connecting the three panels together, and at least two elements (25, 26) connecting the two sides facing each other of the inner panel (2).

10. Implant according to Claim 8, characterized in that the radial elements (4) connect two panels to each other, the third panel (7) being connected to the other two (2, 3) by overstitching its outer edge (5).

11. Implant according to Claim 1, characterized in that it comprises an additional covering panel (9), bound to the textile element (1a), having a shape different from that of the latter and adapted with an appropriate slit for surrounding any anatomical duct.

12. Assembly for obturating an anatomical duct, cavity, or orifice, comprising an obturating implant according to any one of Claims 1 to 11, and an applicator (14).
